Europäisches Patentamt

European Patent Office

Office européen des brevets

(19)

(11) Numéro de publication: **0 214 063**
**A1**

(12)

# DEMANDE DE BREVET EUROPEEN

(21) Numéro de dépôt: 86420179.3

(22) Date de dépôt: 04.07.86

(51) Int. Cl.⁴: **A 61 M 25/00**

(30) Priorité: 08.07.85 FR 8510768

(43) Date de publication de la demande:
11.03.87 Bulletin 87/11

(84) Etats contractants désignés:
AT BE CH DE GB IT LI LU NL SE

(71) Demandeur: **Bazenet, Jean Pierre**
**77 avenue de l'Eygala Corenc**
**F-38700 La Tronche (FR)**

**Chevallier, Jacques**
**27 rue Emile Fontanier**
**F-78320 Le-Mesnil-Saint-Denis (FR)**

(72) Inventeur: **Bazenet, Jean-Pierre**
**77 avenue de l'Eygala**
**Corenc Isère (FR)**

(74) Mandataire: **Maureau, Philippe et al**
**Cabinet Germain & Maureau Le Britannia - Tour C 20, bld**
**Eugène Déruelle**
**F-69003 Lyon (FR)**

Le titre de l'invention a tmodifi(Directives relatives l'examen pratiqul'OEB, A-III, 7.3)

(54) **Sonde intracorporelle et dispositif de montage des ballonnets d'une telle sonde.**

(57) Cette sonde est formée d'un tube souple formant gaine (13) muni à une extrémité d'un ballonnet (3) gonflable à l'intérieur duquel est logé un dispositif de mesure (4), le tube (13) étant muni à son autre extrémité d'une poignée (6) permettant de faire tourner le dispositif de mesure (4) au moyen d'un flexible de commande (2) associé ; elle comporte à son extrémité située du côté du ballonnet (3), un embout (5) de forme fuselée et en matériau souple.

L'embout (5) et le ballonnet (3) sont en silicone de dureté comprise entre 50 et 100 shore et peuvent être réalisés en une seule pièce par moulage par injection.

L'embout (5) présente une partie (8) de forme conique et de pente égale à environ 10 %.

Des paliers (40,7a) sont prévus pour le montage rotatif du support (4) dans le tube (13) et dans l'embout (5).

FIG.1

**Description**

## SONDE INTRACORPORELLE DE TYPE SONDE OESOPHAGIENNE ET DISPOSITIF DE MONTAGE DES BALLONNETS D'UNE TELLE SONDE

La présente invention concerne une sonde intracorporelle destinée par exemple à être introduite à l'intérieur de l'oesophage pour la mesure du débit aortique.

Une sonde de ce type est déjà connue par la demande de brevet français 78 14494 ainsi que par l'article "Mesure du débit aortique par écho Doppler et sonde intraoesophagienne" paru dans le "Journal Français d'Echographie" d'octobre 1983.

Cette sonde est formée d'un tube souple muni à une extrémité d'un ballonnet gonflable en latex à l'intérieur duquel sont placés un ou plusieurs transducteurs ultrasonores montés sur un support et alimentés en courant électrique à l'aide d'un câble logé à l'intérieur du tube souple formant gaine.

La sonde est introduite dans l'oesophage par voie nasale ou buccale, lorsqu'elle est en position dans celui-ci le ballonnet est gonflé par injection d'eau de façon à assurer le couplage ultrasonique · et à immobiliser la sonde dans l'oesophage.

Un flexible introduit à l'intérieur du tube souple, autour duquel est enroulé le câble d'alimentation électrique, et qui est fixé au support des transducteurs permet de faire tourner de l'extérieur les transducteurs à l'intérieur du ballonnet de façon à obtenir l'orientation optimale de ceux-ci pour une mesure correcte.

Une telle sonde, si elle donne satisfaction, présente néanmoins de nombreux inconvénients ayant limité jusqu'à présent son utilisation :
-son introduction dans l'oesophage notamment par la voie nasale est malaisée et difficile du fait que l'extrémité de la sonde qui est formée par le support des tranducteurs est rigide et des risques de perforation oesophagienne sont liées à la forme de cette extrémité,
-le ballonnet en latex doit être extrêmement fin pour ne pas perturber la mesure au niveau des tranducteurs et est donc essentiellement fragile,
-lors de la rotation des transducteurs pour la mise en place de la sonde, les câbles électriques enroulés autour du flexible assurant cette rotation frottent contre le tube souple, ce qui peut provoquer des ruptures de ces câbles.

En outre une telle sonde est d'un prix de revient extrêmement élevé, or elle ne peut être utilisée qu'un nombre de fois limité (tout au plus une dizaine de fois) du fait de la fragilité intrinsèque des ballonnets et son utilisation est donc très coûteuse.

Le but de la présente invention est de remédier à ces inconvénients et notamment de fournir une sonde pouvant être réutilisée pratiquement indéfiniment même lorsqu'il se produit une rupture du ballonnet, qui soit moins coûteuse. qui soit d'utilisation plus facile et plus simple. qui ne présente aucun risque de détérioration lors de la rotation du ballonnet et qui puisse être introduite dans l'oesophage par la voie nasale. cette dernière possibilité étant fondamentale puisqu'une telle sonde est destinée à être utilisée sur des patients qui sont généralement déjà "intubés".

Ce but est atteint en ce que la sonde est munie à son extrémité destinée à être introduite en premier dans le corps d'un embout en un materiau souple et bien toléré par l'organisme et de forme fuselée.

Cet embout peut être en latex, silicone...etc ; il est de préférence en silicone d'une dureté comprise entre 50 et 100 shore.

Ces dispositions permettent une introduction facile de la sonde par voie nasale sans risque de perforation oesophagienne.

Des moyens sont également prévus pour permettre la rotation du support de transducteurs par rapport à l'embout, afin de permettre le réglage azimutal de ceux-ci.

Avantageusement aussi, le ballonnet est en silicone injecté et présente une variation d'épaisseur de façon à assurer à la fois le maintien de ce ballonnet sur le tube et l'embout de la sonde et un bon couplage des ondes ultrasonores.

Un dispositif est également prévu pour permettre le remplacement du ballonet une fois que celui-ci est endommagé et donc pour permettre la réutilisation de la sonde.

De toute façon, l'invention sera bien comprise et d'autres caractéristiques seront mises en évidence à l'aide de la description qui suit en référence au dessin schématique annexé représentant à titre d'exemple non limitatif une forme de réalisation préférée de cette sonde intracorporelle:
- Figure 1 est une vue de dessus de la sonde oesophagienne ;
- Figure 2 est, à échelle agrandie, une vue de détail du montage rotatif du support de transducteurs dans l'embout ;
- Figure 3 est une vue similaire à figure 2 selon une autre forme de réalisation ;
- Figure 4 est, à échelle agrandie, une vue de détail du ballonnet ;
- Figure 5 est une vue en coupe longitudinale du dispositif de montage des ballonnets ;
- Figure 6 à 9 sont des vues schématiques illustrant les différentes étapes de montage d'un ballonnet ;
- Figure 10 est, à échelle agrandie, une vue de détail du tube flexible ;
- Figure 11 est une vue d'un ballonnet et de l'embout selon une seconde forme de réalisation ;
- Figure 12 est une vue similaire à figure 5 du dispositif pour le montage des ballonnets selon la second forme de réalisation ;
- Figure 13 est une vue, en coupe longitudinale, montrant la réalisation du montage rotatif du flexible à l'intérieur de la gaine ;
- Figures 14 à 16 sont des vues de détail, en bout, montrant différentes formes de réalisation du palier de la figure 13.

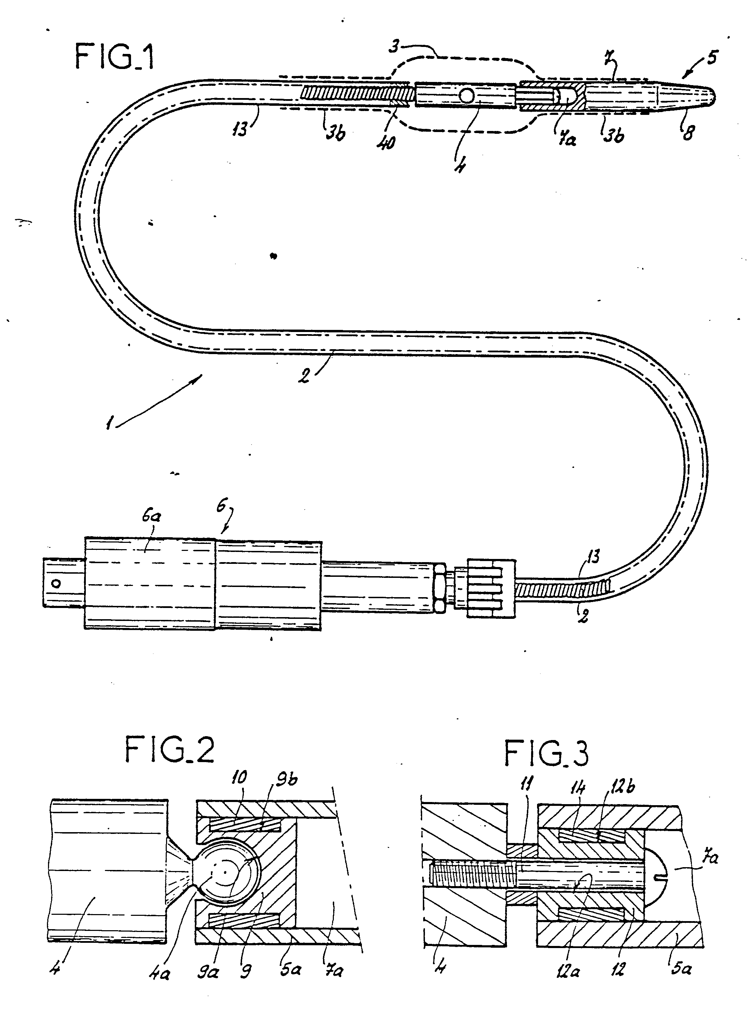

Ainsi que le montre la figure 1, la sonde oesophagienne (1) selon l'invention comprend un tube souple ou flexible (2) logé à l'intérieur d'une gaine de protection (13) en vynil ou en silicone. et qui est fixé par ses extrémités, d'une part, à un support (4) pour le dispositif de mesure et, d'autre part, à une partie (6a) rotative d'une poignée (6) dont l'actionnement permet de faire tourner le support du dispositif de mesure (4) dans un sens ou dans l'autre.

L'extrémité de la sonde (1) destinée à être introduite dans le corps est munie d'un embout (5), et un ballonnet (3) est également prévu autour du dispositif de mesure et de son support (4).

Dans l'exemple de sonde montré dans le dessin, le dispositif de mesure est formé par des transducteurs ultrasonores pour la mesure du débit par effet Doppler, montés sur un support, mais il va de soi que ce dispositif de mesure pourrait être formé tout autrement, afin de mesurer d'autres grandeurs notamment dans le cas de sondes autres qu'oesophagiennes.

L'embout (5) a une forme fuselée et présente une partie cylindrique (7) et une partie conique (8).

La partie conique a une pente d'environ 10 % et est conçue de façon à permettre le guidage de la sonde et à faciliter son passage, par le conduit nasal puis par le passage du pharynx, lors de sa mise en place.

Cet embout (5) est également en un matériau souple et non-agressif de façon à éliminer tout risque de perforation de l'oesophage. Il est donc formé, de préférence, en latex ou en silicone, un matériau préféré étant un silicone de dureté comprise entre 50 et 100 shore.

La partie cylindrique (7) de l'embout (5) est prévue pour permettre le montage d'une des extrémités (3b) du ballonnet (3). Elle est également destinée à permettre son montage rotatif sur le support du dispositif de mesure (4) et elle présente à cet effet un alésage cylindrique (7a), ce qui permet d'avoir un montage rotatif sans saillie extérieure sur la sonde (1).

Les figures 2 et 3 illustrent deux possibilités de réalisation du montage rotatif de cet embout (5).

Dans le cas de la figure 2, le support du dispositif de mesure (4) est muni à son extrémité destinée à être engagée dans l'embout (5) d'une rotule sphérique (4a). Cette rotule (4a) est apte à être introduite par encliquetage dans une cavité sphérique (9a) ménagée dans une bague cylindrique (9) en acier inoxydable montée dans l'alésage cylindrique (7a) de l'embout (5). Cette bague cylindrique (9) présente également sur sa périphérie une gorge annulaire (9b) apte à recevoir une bague intermédiaire en silicone (10). L'ensemble bague (9) et bague intermédiaire (10) est introduit dans l'alésage associé de la partie cylindrique (7) de l'embout (5), la fixation de ces différents éléments étant assurée par le collage de la bague (10) en silicone sur la partie correspondante de l'embout (5) également en silicone. En effet, sans l'interposition de la bague intermédiaire (10), la bague (9) ne pourrait pas être fixée à l'intérieur de l'embout (5), du fait qu'elle n'est pas en silicone comme celui-ci.

Dans le cas de la figure 3, le support du dispositif de mesure (4) ne présente pas de rotule à son extrémité mais est, au contraire, muni d'une vis (11). Cette vis (11) est montée rotative dans l'alésage (12a) d'un palier (12) en acier inoxydable logé à l'intérieur de l'alésage cylindrique (7a) de l'embout (5). De même que dans le cas précédent, le palier (12) présente une gorge (12b) pour le logement d'une bague intermédiaire (14) en silicone permettant d'assurer la fixation du palier (12) à l'embout (5), par collage de cette bague (14) en silicone dans l'alésage (7a) de cet embout (5) en silicone.

Ces deux possibilités de montage rotatif de l'embout (5) sur le support du dispositif (4) permettent d'assurer la rotation de ce support par rapport à l'embout (5) tout en garantissant une bonne tenue lors de l'extraction de la sonde, puisqu'il y a, à chaque fois, outre la liaison par collage entre la bague (10,14) et l'embout (5), une liaison positive entre le support (4) et l'embout (5), soit par liaison de forme (rotule (4a)), soit par vissage (vis (11)).

En outre, tous les éléments employés dans ce système de montage sont en matériau inoxydable (acier inoxydable ou silicone) et non susceptible de provoquer des traumatismes en cas d'arrachement.

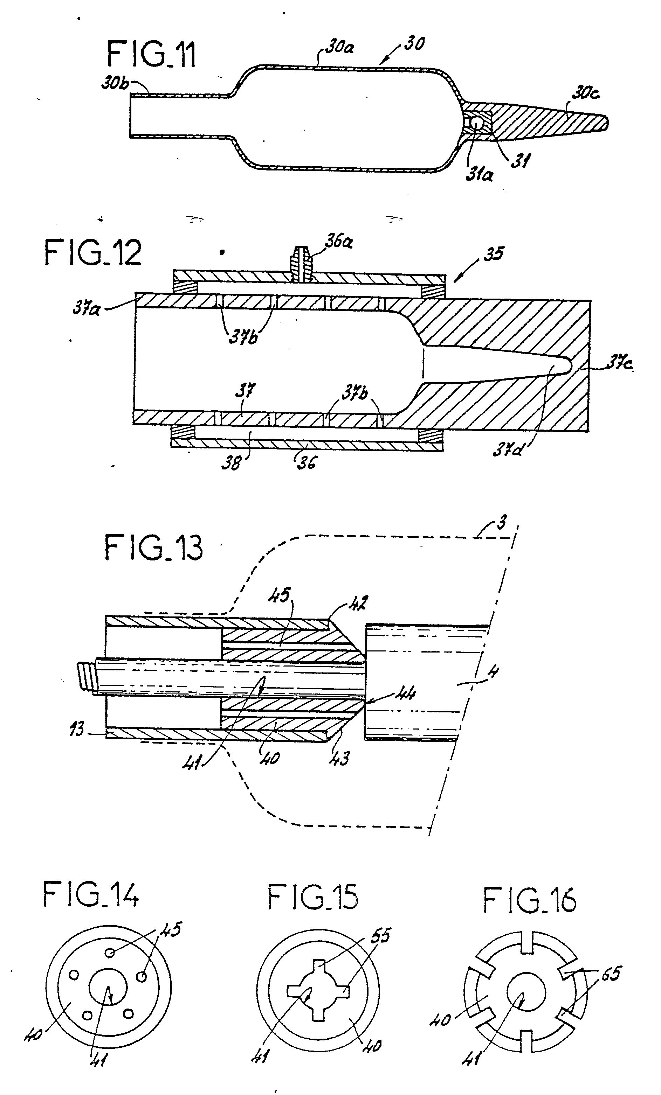

Un autre palier pour le montage rotatif du flexible (2) à l'intérieur de la gaine de protection (13) est également représenté plus en détail sur la figure 13.

Ce palier est formé par un manchon (40) de forme essentiellement cylindrique. Ce manchon (40) est muni d'un alésage concentrique (41) de diamètre supérieur au diamètre extérieur du flexible (2) de façon à en permettre la rotation.

Le diamètre extérieur de ce manchon (40) est par contre légèrement supérieur au diamètre intérieur de la gaine (13) de façon à permettre l'engagement à force de celle-ci sur ce manchon assurant une bonne liaison.

Ce manchon (40) est muni à son extrémité située du côté du support (4) du dispositif de mesure d'un épaulement (42). Cet épaulement (42) fait saillie radialement d'une distance correspondant à l'épaisseur de la gaine (13), de façon à éviter toute saillie par rapport à celle-ci. Cet épaulement (42) est destiné à servir de butée pour cette gaine (13) lors de son introduction sur le manchon (40).

L'épaulement (42) se raccorde à l'extrémité correspondante du manchon (40) par une partie (43) qui peut être conique comme dans l'exemple de la figure ou de forme ovoïde de façon à éviter une agression de l'oesophage par la gaine (13) en cas de déchirement du ballonnet.

Cette partie (43) se termine par une portée arrondie (44) apte à servir de pivot pour le support (4).

Des passages longitudinaux (45) sont également prévus dans le manchon (40) qu'ils traversent de part en part.

Ces passages (45) sont destinés à assurer le passage de l'eau pour le gonflage du ballonnet (3) ou (30).

Le palier (40) ainsi réalisé permet donc d'assurer une bonne concentricité du flexible (2) dans la gaine (13) ainsi qu'un bon fonctionnement en rotation. Bien entendu, il est en un matériau inoxydable tel que de l'acier inoxydable.

Les passages longitudinaux (45) peuvent être formé par des perçages (45) ménagés parallèlement à l'alésage (41) comme montré à la figure 14. Ils peuvent être également formés par des rainures longitudinales intérieures (55) ménagées sur la périphérie de l'alésage (41) et s'étendant parallèlement à celui-ci, en formant un profil crénelé intérieurement comme montré à la figure 15, ou encore par des rainures longitudinales (65) ménagées sur la périphérie extérieure du manchon (40) en formant un profil crénelé extérieurement comme montré à la figure 16.

Ces passages (45) peuvent également être formés par des lumières de forme quelconque, en arc de cercle par exemple.

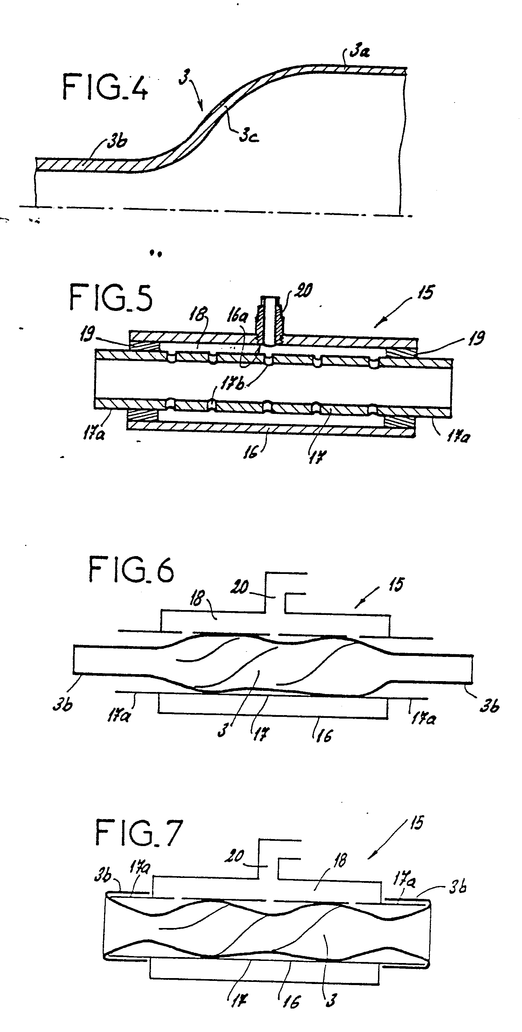

La figure 4 montre un détail de réalisation du ballonnet (3) qui est monté à la fois à l'extrémité de la gaine (13) et sur la partie cylindrique (7) de l'embout (5).

Ce ballonnet (3) présente une partie centrale (3a), formant le ballonnet proprement dit, et deux manchettes (3b) coaxiales de diamètre plus petit prévues à chaque extrémité de la partie centrale (3a) du ballonnet (3) pour son montage sur la gaine (13) et sur la partie (7) de l'embout (5).

Ce ballonnet (3) est, de même que l'embout (5), en silicone et sa partie centrale (3a), est amincie, de façon à permettre le couplage des ondes ultra-sonores et à former un écran le plus faible possible et afin d'offrir un écho minimal pour la mesure par effet Doppler.

Dans l'exemple du dessin, cette épaisseur est de 0,3 mm mais elle peut varier entre 0,2 et 0,5 mm selon la puissance de l'écho.

Cette partie centrale (3a) amincie sert également à garantir une souplesse maximum du ballonnet (3) en position dégonflée de façon à faciliter l'introduction de la gaine comme on le verra plus loin. Par contre, les manchettes (3b) sont en matériau plus épais et ont une épaisseur de 0,4 mm de façon à assurer un serrage efficace de la partie (tube (2), partie (7) de l'embout (5)) sur laquelle elles sont montées et de façon à garantir l'étanchéité, lorsque le ballonnet est rempli d'eau même avec une légère surpression du liquide.

Ainsi que le montre la figure 4, la partie intermédiaire (3c) entre les manchettes (3b) et la partie centrale (3a) présente une variation d'épaisseur progressive afin qu'il n'y ait pas d'amorce de rupture et afin de faciliter le pliage du ballonnet.

Les figures 5 à 9 montrent le dispositif (15) utilisé pour le montage et/ou le remplacement du ballonnet sur la sonde ainsi que les différentes étapes de montage de ce ballonnet. Ce dispositif de montage (15) est formé de deux tubes (16,17) coaxiaux et montés l'un dans l'autre. Le tube intérieur (17) a une diamètre intérieur supérieur au diamètre extérieur de la sonde (1) et une longueur correspondant sensiblement à celle de la partie centrale (3a) du ballonnet ; il est ouvert à chacune de ses extrémités et il est monté à l'intérieur du tube (16) de façon à délimiter entre ces deux tubes un espace annulaire (18).

Ce tube (17) dépasse du tube (16) plus court à chacune de ses deux extrémités en formant des épaulements (17a). Il est muni de trous (17b) répartis régulièrement sur sa périphérie. Le tube extérieur (16) est monté de façon étanche par interposition de deux bagues (19) autour du tube (17). Il est muni sur la périphérie d'un orifice (16a) à l'intérieur duquel est fixé un raccord (20) apte à être raccordé sur une pompe à vide.

Ces deux tubes (16,17) sont de préférence en un matériau transparent tel que celui connu sous la dénomination commerciale "Altuglass" de façon à bien permettre la visualisation des différentes étapes de montage.

Le montage du ballonnet (3) sur la sonde (2) à l'aide du dispositif de montage (15) a lieu de la façon suivante :

Le ballonnet (3) est tout d'abord introduit en étant replié sur lui-même à l'intérieur du tube (17) du dispositif (15) comme montré à la figure 6.

Les manchettes du ballonnet (3b) sont ensuite rabattues sur les épaulements, formés par les extrémités (17a) du tube (17), du dispositif (15) (Cf. figure 5).

Le raccord (20) du dispositif est alors raccordé à une pompe à vide de façon à faire le vide dans l'espace annulaire (18) ménagé entre les deux tubes (16) et (17).

Sous l'action de la dépression ainsi créée dans l'espace intermédiaire (18) le ballonnet se trouve plaqué contre les parois du tube central (17) (Cf. Figure 6).

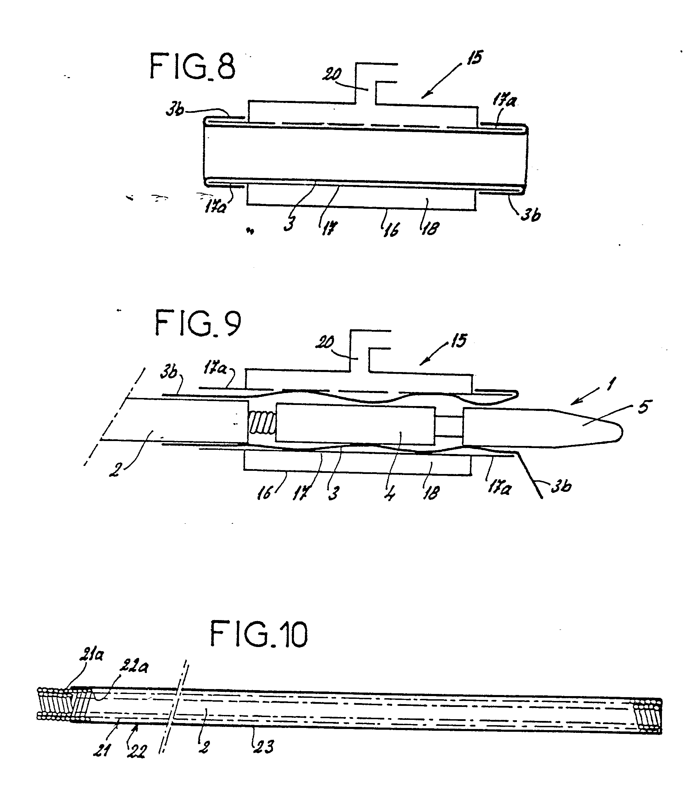

La sonde (1) est alors introduite à l'intérieur du dispositif (15) et positionnée correctement de façon que le dispositif de mesure (4) se trouve à l'intérieur du ballonnet (3) et que les extrémités du tube (2) et de l'embout (5) se trouvent au niveau des épaulements (17a) du dispositif. Il suffit ensuite de remettre la cavité (18) à la pression atmosphérique en réintroduisant de l'air à l'intérieur de celle-ci par le raccord (20) et de rabattre la partie repliée des deux manchettes du ballonnet sur le tube (2) et sur l'embout (5) comme montré à la figure 9, pour obtenir le montage du ballonnet (3) sur la sonde (1). La sonde peut alors être retirée du dispositif de montage (15).

La figure 11 montre un autre exemple de réalisation du ballonnet (30). Das ce cas, le ballonnet (30) présente également une partie centrale (30a) formant le ballonnet proprement dit et une manchette (30b) coaxiale formée à une extrémité de cette partie centrale (30a). Cependant, l'autre extrémité (30c) n'a pas la forme d'une manchette mais celle d'un embout de forme fuselée similaire à celle de l'embout (5).

Dans ce cas également, l'embout (30c) ne présente pas de partie cylindrique et seulement une partie conique puisqu'il fait partie du ballonnet (30).

Le ballonnet (30) et l'embout (30c) sont réalisés simultanément en silicone par moulage par injection.

Le palier pour le montage rotatif du support (4) est alors formé par une bague cylindrique (31) similaire à la bague (9) précédemment décrite et munie comme celle-ci d'une cavité sphérique (31a). Cette bague (31) est surmoulée en même temps que le ballonnet (30) et peut présenter sur sa périphérie des rainures ayant par exemple la forme de queues d'arondes pour assurer une liaison de forme entre cette bague et la partie (30c) du ballonnet, une fois le moulage terminé.

Lors du montage de la sonde, le support (4) sera simplement encliqueté par sa partie (4a) comme expliqué précédemment, dans la cavité sphérique (31a) de la bague (31).

La figure. 12 montre la forme de réalisation du dispositif de montage (35) du ballonnet (30).

Dans ce cas, de même que pour le dispositif (15), le dispositif (35) est également formé de deux tubes (36,37) coaxiaux montés l'un dans l'autre, délimitant entre eux un espace annulaire (38), des trous (37b) étant répartis régulièrement sur la périphérie du tube (37). Cependant, dans ce cas, le dispositif n'est ouvert et muni d'un épaulement qu'à une extrémité, son autre extrémité (37c) étant, au contraire, fermée et munie d'un évidement (37d) de forme complémentaire de celle de la partie d'embout (30c) du ballonnet (30) de façon à accueillir celui-ci.

Le montage du ballonnet (30) s'effectue de la même façon que décrite précédemment pour le ballonnet (3), par application d'une dépression par l'orifice (36a) du tube (36), mis à part que dans ce cas une seule manchette (30b) est retournée sur l'épaulement (37a).

La figure 10 montre le mode de réalisation du tube souple (2).

Celui-ci est formé de deux ressorts (21,22) enroulés à des pas inverses l'un de l'autre et disposés l'un dans l'autre de façon que leurs spires (21a,22a) se croisent. L'ensemble est, en outre, muni d'une enveloppe extérieure (23) pouvant être formée soit par une gaine extérieure souple thermorétractable en silicone soit par une projection de silicone à haute température.

Le double enroulement du tube (2) permet d'empêcher que le tube (2) se torde ou se resserre, lors de la rotation de la poignée (6), au lieu de transmettre ce mouvement de rotation au dispositif de mesure (4). L'enveloppe (23) permet de rigidifier les deux ressorts (21) et (22) en rotation et d'éviter que leurs spires (21a) et (22a) ne s'ouvrent lors du dévissage.

En outre, comme ce tube(2) est creux, les câbles d'alimentation (non représentés sur le dessin) du dispositif de mesure (4) peuvent être logés à l'intérieur de celui-ci et ne risquent donc pas de se tordre et de s'abimer lors de la rotation du dispositif de mesure (5), puisqu'ils ne tournent pas, comme dans les dispositifs connus jusqu'à présent où le câble de commande logé à l'intérieur du tube souple et où les câbles d'alimentation sont enroulés autour de ce câble de commande.

On peut noter que la sonde obtenue à l'aide de l'invention est formée uniquement de matériaux inoxydables et bien tolérés par l'organisme.

Elle ne présente, en outre, extérieurement, ni aspérité (au niveau du montage du ballonnet et de l'embout), ni partie métallique susceptible d'endommager l'oesophage dans lequel elle est introduite.

Enfin, grâce au dispositif de montage des ballonnets, elle peut être réutilisée un très grand nombre de fois, même en cas de rupture des ballonnets, ce qui en diminue considérablement le prix de revient.

Comme il va de soi, la présente invention ne se limite pas à la seule forme d'exécution décrite ci-avant à titre d'exemple non limitatif ; elle en embrasse, au contraire, toutes les formes de réalisation mettant en oeuvre des moyens similaires ou équivalents.

Notamment, les différentes dispositions constructives montrées ci-avant peuvent être utilisées pour n'importe quel type de sonde intracorporelle et notamment pour des sondes autres qu'oesophagiennes ou destinées à des mesures autres que le débit aortique, ou ne comportant pas de dispositif de mesure à l'intérieur du ballonnet.

## Revendications

1- Sonde intracorporelle du type formée d'un tube souple formant gaine (13) muni à une extrémité d'un ballonnet (3) gonflable à l'intérieur duquel est logé un dispositif de mesure (4), le tube (13) étant muni à son autre extrémité d'une poignée (6) permettant de faire tourner le dispositif de mesure (4) au moyen d'un flexible de commande (2) associé, caractérisée en ce qu'elle comporte à son extrémité située du côté du ballonnet (3,30), un embout (5, 30c) de forme fuselée et en matériau souple.

2- Sonde intracorporelle selon la revendication 1, caractérisée en ce que l'embout (5, 30c) et le ballonnet (3,30) sont en silicone de dureté comprise entre 50 et 100 shore.

3- Sonde intracorporelle selon l'une des revendications 1 ou 2, caractérisée en ce que l'embout (30c) est réalisé en une seule pièce avec le ballonnet (30) par moulage par injection.

4- Sonde intracorporelle selon l'une des revendications 1 à 3, caractérisée en ce que l'embout (5,30c) présente une partie (8,30c) de forme conique et de pente égale à environ 10 %.

5- Sonde intracorporelle selon l'une quelconque des revendications 1 à 4, caractérisée en ce que l'embout (5) est muni d'un alésage (3a) apte à recevoir un palier (9,12) pour le montage rotatif du support du dispositif de mesure.

6- Sonde intracorporelle selon la revendication 5, caractérisée en ce que le palier (9,12) est muni extérieurement d'une gorge périphérique apte à recevoir une bague intermédiaire (10,14) en silicone qui est apte à être fixée par collage à l'intérieur de l'alésage (7a) de l'embout (5).

7- Sonde intracorporelle selon la revendication 5, lorsque celle-ci est rattachée à la revendication 3, caractérisée en ce que le palier (31) est surmoulé à l'intérieur de l'embout (30c) du ballonnet (30).

8- Sonde intracorporelle selon l'une des revendications 5 à 7, caractérisée en ce que le support du dispositif de mesure (4) est muni à son extrémité destinée à être engagée dans l'embout (5,30c) d'une rotule sphérique (4a) apte à être introduite par encliquetage dans une cavité sphérique (9a,31a) ménagée dans la bague (9,31) formant palier, montée à l'intérieur de l'embout (5,30).

9- Sonde intracorporelle selon l'une quelconque des revendica tions précédentes, caractérisée en ce qu'un palier (40) est prévu à l'extrémité de la gaine souple (13) pour le montage rotatif du flexible (2) à l'intérieur de celle-ci et en ce que ce palier (40) est muni de passages longitudinaux (45) aptes à laisser passer l'eau pour le remplissaage du ballonnet (3,30).

10- Sonde intracorporelle selon la revendication 9, caractérisée en ce que le palier (40) est muni d'un épaulement (42) et en ce qu'il se termine par une partie (43) conique ou de forme ovoïde.

11- Sonde intracorporelle selon l'une quelconque des revendications précédentes, caractérisée en ce que le tube souple (2) est formé de deux ressorts (21,22) enroulés à des pas inverses l'un de l'autre et disposés l'un dans l'autre de façon que leurs spires (21a,22a) se croisent, l'ensemble étant muni d'une enveloppe extérieure (23) pouvant être formée soit par une gaine extérieure souple thermorétractable en silicone, soit par une projection de silicone à haute température.

12- Sonde intracorporelle selon l'une quelconque des revendications précédentes, caractérisée en ce que le ballonnet (3,30) présente une partie centrale (3a,30a) formant le ballonnet proprement dit et de faible épaisseur et au moins un manchette (3b,30b) coaxiale de diamètre plus petit et d'épaisseur plus grande.

13- Sonde intracorporelle selon la revendication 10, caractérisée en ce que l'épaisseur de la partie centrale du ballonnet (3,30) varie entre 0,2 et 0,5 mm.

14- Dispositif de montage des ballonnets d'une sonde selon l'une quelconque des revendications précédentes, caractérisé en ce qu'il comporte deux tubes (16,17 ;36,37) coaxiaux montés l'un dans l'autre et délimitant entre eux un espace annulaire (18,38), le tube intérieur (17,37) étant muni de trous (17b,37b) sur sa périphérie et faisant saillie par rapport au tube extérieur (16,36) en formant au moins un épaulement (17a, 37a), et le tube extérieur (16,36) étant apte à être raccordé à une pompe à vide, en ce que le tube intérieur (17,37) a un diamètre intérieur supérieur à celui de la sonde (1) et une longueur correspondant sensiblement à celle de la partie médiane (3a) d'un ballonnet (3,30), en ce que chaque ballonnet (3,30) est apte à être introduit dans le tube (17,37) du dispositif et à y être plaqué contre ses parois par application de vide dans l'espace annulaire (18,38), de façon à permettre l'introduction de la sonde (2) à l'intérieur du ballonnet (3,30) et en ce que chaque ballonnet est apte à être ensuite plaqué contre la sonde (2) par élimination du vide établi dans l'espace annulaire (18,38).

15- Dispositif de montage selon la revendication 14, caractérisé en ce que, dans le cas où le ballonnet (30) est solidaire de l'embout (30c), le dispositif présente un évidement (37d) de forme complémentaire de celle de la partie formant embout (30c) du ballonnet (30).

86420179 3

**FIG.1**

**FIG.2**

**FIG.3**

FIG.4

3a

3

3c

3b

FIG.5

20

18    16a    15

19    19

17b

17a    17a

16    17

FIG.6

18    20    15

3b    3b

17a    17a

3    17    16

FIG.7

15

20    18

3b    17a    17a    3b

17    16    3

## FIG.8

## FIG.9

## FIG.10

FIG. 11

30b 30a 30 30c
31a 31

FIG. 12

36a 35
37a
37b
37c
37 37b
38 36 37d

FIG. 13

3
45 42
4
44
13 41 40 43

FIG. 14          FIG. 15          FIG. 16

45              55              65
40              40              40
41              41   40         41

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int Cl 4) |
|---|---|---|---|
| Y | FR-A-2 424 733  (INSERM)<br><br>* Figures 1-3; page 2, ligne 34 - page 4, ligne 29 * | 1,2,5, 11 | A 61 M  25/00 |
| Y | US-A-3 926 705  (TODD)<br><br>* Figures 1,2; colonne 4, ligne 4 - colonne 5, ligne 40 * | 1,2,5, 11 | |
| A | | 14 | |
| Y | US-A-3 749 086  (KLINE et al.)<br>* Figures 1,3; colonne 2, lignes 55-66; colonne 3, lignes 10-14 * | 11 | |
| A | US-A-3 773 034  (BARNS et al.) | | **DOMAINES TECHNIQUES RECHERCHES (Int. Cl.4)**<br><br>A 61 M |
| A | DE-B-1 491 793  (AMERICAN HOSPITAL SUPPLY)<br><br>----- | | |

Le present rapport de recherche a ete etabli pour toutes les revendications

| Lieu de la recherche<br>LA HAYE | Date d achevement de la recherche<br>23-09-1986 | Examinateur<br>DEUTSCH J.P.M. |
|---|---|---|